(19) Europäisches Patentamt
European Patent Office
Office européen des brevets

(11) Veröffentlichungsnummer: **0 484 855 B1**

(12) **EUROPÄISCHE PATENTSCHRIFT**

(45) Veröffentlichungstag der Patentschrift: **14.06.95**

(51) Int. Cl.$^6$: **C07C 255/30**, C07C 253/30

(21) Anmeldenummer: **91118769.8**

(22) Anmeldetag: **04.11.91**

(54) **Verfahren zur Herstellung von 3-Aminocrotonnitril.**

(30) Priorität: **05.11.90 CH 3504/90**

(43) Veröffentlichungstag der Anmeldung:
**13.05.92 Patentblatt 92/20**

(45) Bekanntmachung des Hinweises auf die Patenterteilung:
**14.06.95 Patentblatt 95/24**

(84) Benannte Vertragsstaaten:
**AT BE CH DE DK ES FR GB IT LI NL SE**

(56) Entgegenhaltungen:
**AT-A- 375 067**
**CH-A- 415 608**
**US-A- 3 290 355**

**JOURNAL OF THE AMERICAN CHEMICAL SOCIETY Bd. 64, 1942, WASHINGTON D.C. Seiten 150 - 154; H ADKINS ET AL: 'Hydrogenation of beta-iminonitriles'**

(73) Patentinhaber: **LONZA AG**
**(Geschäftsleitung: 4002 Basel)**
**CH-3945 Gampel/Wallis (CH)**

(72) Erfinder: **Ouittmann, Wilhelm, Dr.**
**Rottenstrasse 3**
**Visp (Kanton Wallis) (CH)**
Erfinder: **Zumoberhaus, Leo**
**Haus Abendruh**
**Bürchen (Kanton Wallis) (CH)**
Erfinder: **Ruppen, Peter, Dr.**
**Mühlackerstrasse 1**
**Visp (Kanton Wallis) (CH)**

(74) Vertreter: **Weinhold, Peter, Dr. et al**
**Patentanwälte**
**Dr. Weinhold, Dannenberg,**
**Dr. Gudel, Schubert**
**Siegfriedstrasse 8**
**D-80803 München (DE)**

**EP 0 484 855 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung beinhaltet ein neues Verfahren zur Herstellung von 3-Aminocrotonnitril durch Dimerisierung von Acetonitril.

3-Aminocrotonnitril ist ein Zwischenprodukt mit sehr breitem Anwendungsspektrum. So findet es z.B. seinen Einsatz zur Herstellung von Pharmazeutika CA 85, 177262, zur Herstellung von Pestiziden CA 79, 53185b oder zur Herstellung von Azofarbstoffen CA 80, 122383.

Es sind zahlreiche Verfahren bekannt, um 3-Aminocrotonnitril über die Dimerisierung von Acetonitril in Gegenwart von starken Basen herzustellen.
Problematisch bei dieser Reaktion ist aber, dass Acetonitril mit einer Base, wie z.B. einem Amid-Ion auf verschiedenen Weise reagieren kann.

Wird nämlich die Nitrilgruppe angegriffen, bildet sich mit dem Amid-Ion das unerwünschte Acetamidin. Lediglich die Deprotonierung des Acetonitrils zum Carbanion bildet die Voraussetzung zur Herstellung des gewünschten 3-Aminocrotonnitrils.

So resultierte gemäss Takeda et al. J.Pharm.Chem.Soc., Japan 75, S.957-959 (1955) nach Umsetzung von Acetonitril mit Natriumamid bei 100 °C während 3 Stunden ein Gemisch von 3-Aminocrotonnitril und Acetamidin.

In der Folge wurde versucht, durch gezielte Reaktionsführung, u.a. durch die Anwendung von sterisch anspruchsvollen, starken Basen, die Carbanionbildung zu begünstigen.

Aufbauend auf Untersuchungen von Ziegler et al., Justus Liebigs Annalen 504, 115 (1933), worin Acetonitril mit Lithiumdiethylamid als Base gezielt in 86% Ausbeute zum 3-Aminocrotonnitril (Iminonitril) umgesetzt wurde, hat Krüger, J.Organomet. Chem; 9, S.125-134 (1967) mit Natrium-bis-trimethylsilylamid in 90% Ausbeute das 3-Aminocrotonnitril synthetisiert. Von den recht guten Ausbeuten abgesehen, haben die genannten Synthesen den Nachteil, dass die sterisch anspruchsvollen Basen z.T. aufwendig und insbesondere teuer hergestellt werden müssen, was für die Umsetzung dieser Verfahren in den Grossbetrieb aus ökonomischen Gründen ein Hindernis darstellt. Eigene Vergleichsbeispiele sollen dies verdeutlichen.

Es ist aus der CH-PS 415 603 aber auch bekannt, Acetonitril mit Hilfe von Natrium in einem aliphatischen Kohlenwasserstoff als Lösungsmittel bei 70-180 °C in einer Ausbeute von 90% zum 3-Aminocrotonnitril zu kondensieren. Diese Dimerisierung hat den Nachteil, dass, bedingt durch den Reaktionsmechanismus, 3 Mole Acetonitril bezogen auf 2 Mole Natrium anstatt 2 Mole Acetonitril bezogen auf 1 Mol Natrium bzw. Base verwendet werden müssen.

Obschon dieser Prozess ein Betriebsverfahren darstellt, ist er aus den genannten Gründen unökonomisch und durch den Anfall von Natriumcyanid auch unökologisch.

Es bestand daher die Aufgabe, ein Verfahren zu entwickeln, das die genannten Nachteile ausschliesst und mit dem man in der Lage ist, 3-Aminocrotonnitril ökologisch und ökonomisch vertretbar im Grossmassstab zu produzieren.

Die Aufgabe konnte mit einem Verfahren nach Patentanspruch 1 gelöst werden.

Die Dimerisierung von Acetonitril, gemäss dem erfindungsgemässen Verfahren erfolgt nach dem Mechanismus

a) Deprotonierung des Acetonitrils in Gegenwart einer starken Base gemäss

$NaNH_2 + CH_3CN \rightarrow Na^{\oplus}CH_2^{\ominus}CN + NH_3$

b) Bildung des Natriumsalzes von 3-Aminocrotonnitril, durch Reaktion des Acetonitrilcarbanions mit Acetonitril gemäss

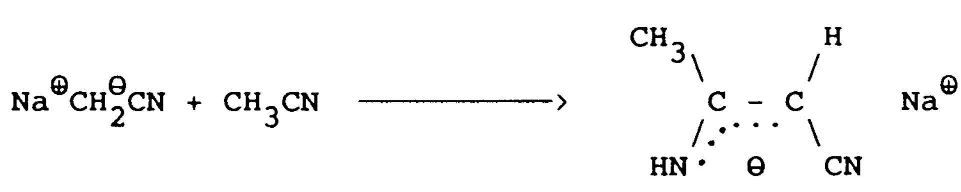

c) und durch Hydrolyse des Natriumsalzes von 3-Aminocrotonnitril mit Wasser gemäss

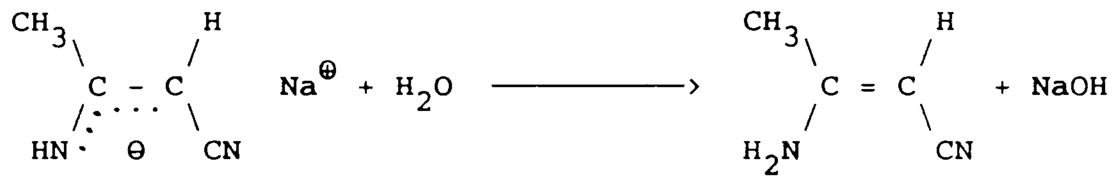

Erfindungsgemäss wird die Deprotonierung des Acetonitrils mit Natriumamid als starker Base,welches zweckmässig zuvor in situ und auf bekannte Weise (F.W. Bergström, W.C. Frenelius, Chem. Rev 12, 45ff. (1933)) durch katalytische Umsetzung von Natrium mit flüssigem Ammoniak hergestellt wird, in Gegenwart von flüssigem Ammoniak durchgeführt.
Zweckmässig erfolgt die Deprotonierung in dem Temperaturbereich, bei dem das Ammoniak bei Normaldruck in flüssigem Aggregatszustand vorliegt, d.h. zwischen -32°C und -75°C. Vorzugsweise erfolgt die Deprotonierung bei einer Temperatur zwischen -32°C bis -35°C.

Das bei der Deprotonierung freiwerdende und das überschüssige als Lösungsmittel verwendete Ammoniak wird zweckmässig recycliert und für die Herstellung des Natriumamids eingesetzt.

Von Vorteil wird so vorgegangen, dass zum in situ hergestellten Natriumamid, welches in einem Überschuss an flüssigem Ammoniak vorliegt, das Acetonitril zugegeben wird.

Dabei ist zweckmässig ein Molverhältnis Acetonitril zu Natriumamid von 2 zu 1 einzuhalten.
Zweckmässig wird das Acetonitril bereits im für die nachfolgende Salzbildung benötigten inerten Lösungsmittel, vorzugsweise in Toluol, der Natriumamidlösung zugegeben.

Als inerte Lösungsmittel sind anstelle von Toluol weitere aromatische Kohlenwasserstoffe, wie z.B. Xylol oder Benzol, Ether, wie z.B. Tetrahydrofuran oder Dimethoxyethan, oder Amine, wie z.B. aliphatische Di- oder Tri-alkylamine, verwendbar.

Die Deprotonierung erfolgt rasch, so dass zweckmässig unmittelbar nach der Zugabe auf die für die Bildung des Natriumsalzes des 3-Aminocrotonnitril erforderliche Temperatur von -5°C bis +35°C, vorzugsweise auf Raumtemperatur, erwärmt werden kann.
Dabei wird das sich verflüchtigende Ammoniak aufgefangen und recycliert.
Nach üblicherweise ca. 0,5 h bis 2 h ist die Salzbildung abgeschlossen. Darauf kann durch langsames Eintragen von Wasser zum Reaktionsgemisch das Natriumsalz des 3-Aminocrotonnitrils auf bekannte Weise, z.B. gemäss CH-PS 415 603, hydrolysiert und in das 3-Aminocrotonnitril überführt werden.
Aus dem Reaktionsgemisch kann darauf auf fachmännische Art und Weise, vorzugsweise destillativ, das 3-Aminocrotonnitril in Ausbeuten von über 90% und Reinheiten von über 99,5% erhalten werden.

Beispiel 1

In einem mit trockenem Stickstoff gespülten Doppelmantelrührwerk wurde unter Katalyse von 0,2 g Eisen(III)nitrat aus 13,8 g Natrium (0,6 Mol) das Natriumamid mit 250 ml flüssigem Ammoniak hergestellt.
Innerhalb von 25 min wurde eine Lösung von 49,3 g Acetonitril (1,2 Mol) in 200 ml Toluol bei der Temperatur des flüssigen Ammoniaks (-33°C) zugetropft. Nach beendeter Zugabe wurde das Ammoniak abgedampft.
Nachdem die Suspension durch Erwärmen eine Temperatur von 20°C erreicht hatte, liess man für eine weitere Stunde bei dieser Temperatur nachreagieren und unterwarf das Reaktionsgemisch danach einer langsamen Hydrolyse durch Zutropfen von 75 ml Wasser.
Nach einer Nachreaktion von 15 min unter Rühren wurde das Gemisch zur Phasenseparation in einen Scheidetrichter überführt, wobei sich 3 Phasen ausbildeten.
Nach Abtrennung der unteren wässrigen Phase, die zur Reinigung noch 2 mal mit je 25 ml Lösungsmittel extrahiert wurde, wurden die vereinigten org. Phasen zunächst am Rotationsverdampfer im Wasserstrahlvakuum vom Toluol befreit und anschliessend einer Vakuum-Destillation unterworfen.
Bei einem Vakuum von 20 mbar und einer Temperatur von 134 bis 140°C destillierte 99,7 bis 99,8%iges 3-Aminocrotonnitril über. Die Ausbeute betrug 46,4 g (94,1%).
Das Lösungsmittel Toluol und das Ammoniak wurden wieder in den Prozess recycliert.

Beispiele 2 bis 7

In diesen Beispielen wurde ein anderes inertes Lösungsmittel an Stelle von Toluol verwendet, die Menge der anderen Edukte jedoch konstant gehalten.

| Bsp. | inertes Lösungsmittel | % Ausbeute | % Reinheit |
|---|---|---|---|
| 2 | Xylol, 225 ml (Isomer.-Mischung) | 87,0 | 99,6 |
| 3 | Benzol, 225 ml | 85,8 | 99,7 |
| 4 | Dimethoxyethan (1, 2), 200 ml | 78,3 | 99,6 |
| 5 | Tetrahydrofuran, 200 ml | 87,6 | 99,5 |
| 6 | Diethylamin, 225 ml | 88,2 | 99,5 |
| 7 | Triethylamin, 225 ml | 88,2 | 99,4 |

In allen weiteren Punkten entsprach die Ausführung der Beispiele 2 bis 7 dem Beispiel 1.

Vergleichsbeispiel 1

In einem thermostatisierbaren Doppelmantelrührwerk wurden 24,3 g Diisopropylamin in 100 ml absol. Tetrahydrofuran gelöst und bei einer Temperatur von maximal 20 °C 150 ml einer 0,16 molaren Lösung von n-Butyllithium in Hexan unter Rühren zugetropft.
Nach beendeter Reaktion wurde die Reaktionsmischung zu einer Lösung von 19,7 g (0,48 Mol) Acetonitril in 60 ml Tetrahydrofuran bei maximal -20 °C zudosiert. Man liess nach beendeter Zugabe 30 min bei dieser Temperatur unter Rühren nachreagieren und erwärmte innerhalb 1 h auf 20 °C, wobei sich eine weisse Masse an den Gefässwänden abschied.
Nach Stehen über Nacht wurde das Reaktionsgemisch bei einer Temperatur von 5 bis 7 °C mit einer Lösung von 14,5 g Essigsäure in 50 ml Wasser hydrolysiert.
Nach der Phasentrennung wurde die wässrige Phase noch zweimal mit je 25 ml Ether extrahiert.
Die vereinigten organischen Phasen wurden im Wasserstrahlvakuum durch Destillation vom Lösungsmittel und vom Amin befreit. Destillation unter den gleichen Bedingungen wie in Beispiel 1 erbrachte 17,0 g 3-Aminocrotonnitril (86,3% Ausbeute) mit einer Reinheit von 98,9%.
Das Verfahren liess sich, wie in den Beispielen 2 bis 4 gezeigt wurde, auf weitere sekundäre und silylierte Amine, die in lithiierter Form als Hilfsbase dienten, ausdehnen. Als Metallierungsreagenz wurde eine der Aminmenge äquivalente Lösung von n-Butyllithium (0,16 Mol in Hexan) verwendet.

| Vergl. Bsp. | sekundäres Amin | Lösungsmittel Menge (ml) | Reaktions- ** temperatur (°C) | Ausbeute/Reinheit (%) |
|---|---|---|---|---|
| 2 | N-Methylanilin 0,24 Mol | Tetrahydrofuran (160 ml) | -24 | 60/92,6 (Rohprodukt) |
| 3 | Diethylamin 0,3 Mol | Diethylether (160 ml) | -23 | 56,9/99,0 |
| 4 | $H-N(SiMe_3)_2$ 0,6 Mol | Tetrahydrofuran (75 ml) | 0 | 90,5/99,6 |

** Temperatur der Deprotonierung

Vergleichsbeispiel 5

Mittels eines Dispergierrührwerkes wurden 3,0 g (0,13 Mol) Natriummetall in 115,5 g Hexamethyldisilazan (Überschuss) bei einer Temperatur von 124 °C emulgiert.
Unter der Katalyse von 0,5 g Eisen(III)stearat bildete sich das Natriumsalz von Hexamethyldisilazan innerhalb von 5 h 30 min. Bei einer Temperatur von 80 bis 92 °C wurde Acetonitril (10,7 g = 0,26 Mol) zugetropft und das auf 20 °C abgekühlte Reaktionsgemisch filtriert.
Der Filterkuchen wurde mit 30 ml tert.Butyl-methylether gewaschen und in 100 ml Wasser verrührt.
Nach Phasenseparation wurde die wässrige Phase 2 mal mit 50 ml und 1 mal mit 25 ml Ether extrahiert.
Das Lösungsmittel wurde am Rotationsverdampfer entfernt und der Rückstand im Vakuum destilliert, wobei man 5,7 g 99,5%iges 3-Aminocrotonnitril in einer Ausbeute von 53,3% erhielt.
Aus den Filtraten wurde Hexamethyldisilazan in einer Ausbeute bis zu 95% durch fraktionierte Destillation recycliert.

## Patentansprüche

1. Verfahren zur Herstellung von 3-Aminocrotonnitril durch Dimerisierung von Acetonitril,beinhaltend die Reaktionsschritte Deprotonierung des Acetonitrils in Gegenwart einer starken Base, Salzbildung des 3-Aminocrotonnitrils und Hydrolyse des Salzes,dadurch gekennzeichnet, dass die Deprotonierung mit Natriumamid als starker Base in Gegenwart von flüssigem Ammoniak erfolgt.

2. Verfahren nach Patentanspruch 1, dadurch gekennzeichnet, dass die Deprotonierung bei Normaldruck und bei Temperaturen des flüssigen Ammoniaks zwischen -32 °C und -75 °C erfolgt.

3. Verfahren nach den Patentansprüchen 1 oder 2, dadurch gekennzeichnet, dass für die Dimerisierung ein Molverhältnis Acetonitril zu Natriumamid von 2 zu 1 eingehalten wird.

4. Verfahren nach mindestens einem der Patentansprüche 1 bis 3, dadurch gekennzeichnet, dass die Salzbildung des 3-Aminocrotonnitrils zwischen -5 °C und +35 °C in Gegenwart eines inerten Lösungsmittels erfolgt.

5. Verfahren nach Patentanspruch 4, dadurch gekennzeichnet, dass als inertes Lösungsmittel Toluol verwendet wird.

6. Verfahren nach mindestens einem der Patentansprüche 1 bis 5, dadurch gekennzeichnet, dass die Hydrolyse auf bekannte Weise durch Zugabe von Wasser erfolgt.

7. Verfahren nach mindestens einem der Patentansprüche 1 bis 6, dadurch gekennzeichnet, dass das Natriumamid vor dem Dimerisieren in situ und auf bekannte Weise durch katalytische Umsetzung von Natrium mit flüssigem Ammoniak hergestellt wird.

8. Verfahren nach mindestens einem der Patentansprüche 1 bis 7, dadurch gekennzeichnet, dass die die Dimerisierung beinhaltenden Reaktionsschritte ohne Isolierung der Zwischenstufen durchgeführt werden.

## Claims

1. Process for the preparation of 3-aminocrotononitrile by dimerization of acetonitrile, comprising the reaction steps of deprotonation of the acetonitrile in the presence of a strong base, salification of the 3-aminocrotononitrile and hydrolysis of the salt, characterized in that said deprotonation is effected using sodium amide as the strong base in the presence of liquid ammonia.

2. The process of claim 1, characterized in that the deprotonation is effected at normal pressure and at liquid ammonia temperatures of from -32 °C to -75 °C.

3. The process of claims 1 or 2, characterized in that a molar ratio between acetonitril and sodium amide of 2 to 1 is maintained for dimerization.

4. The process of at least one of claims 1 to 3, characterized in that salification of the 3-aminocrotononitrile is effected at between -5 °C and +35 °C in the presence of an inert solvent.

5. The process of claim 4, characterized in that toluene is used as the inert solvent.

6. The process of at least one of claims 1 to 5, characterized in that hydrolysis is effected in a known manner by the addition of water.

7. The process of at least one of claims 1 to 6, characterized in that the sodium amide is prepared before dimerization in situ and in a known manner by catalytical reaction of sodium with liquid ammonia.

8. The process of at least one of claims 1 to 7, characterized in that the reaction steps comprising the dimerization are carried out without isolation of the intermediates.

## Revendications

1. Procédé pour la préparation de 3-aminocrotononitrile par dimérisation de l'acétonitrile, comprenant les étapes réactionnelles de déprotonisation de l'acétonitrile en présence d'une base forte, formation de sel du 3-aminocrotononitrile et hydrolyse du sel, caractérisé en ce que la déprotonisation s'effectue avec un amide de sodium en tant que base forte en présence d'ammoniaque liquide.

2. Procédé selon la revendication 1, caractérisé en ce que la déprotonisation s'effectue à pression normale et à des températures de l'ammoniaque liquide entre -32 °C et -75 °C.

3. Procédé selon les revendications 1 ou 2, caractérisé en ce que pour la dimérisation, on maintient un rapport molaire entre l'acétonitrile et l'amide de sodium de 2 à 1.

4. Procédé selon au moins l'une des revendications 1 à 3, caractérisé en ce que la formation de sel du 3-aminocrotononitrile s'effectue entre -5 et +35 °C en présence d'un solvant inerte.

5. Procédé selon la revendication 4, caractérisé en ce qu'en tant que solvant inerte, on utilise du toluol.

6. Procédé selon au moins l'une des revendications 1 à 5, caractérisé en ce que l'hydrolyse s'effectue de la manière connue par addition d'eau.

7. Procédé selon au moins l'une des revendications 1 à 6, caractérisé en ce que l'amide de sodium est préparé avant la dimérisation in situ et de la manière connue par réaction catalytique du sodium avec de l'ammoniaque liquide.

8. Procédé selon au moins l'une des revendications 1 à 7, caractérisé en ce que les étapes réactionnelles comportant la dimérisation sont effectuées sans isoler les étapes intermédiaires.